(19) Europäisches Patentamt
European Patent Office
Office européen des brevets

(11) **EP 1 757 308 A1**

(12) **EUROPEAN PATENT APPLICATION**
published in accordance with Art. 158(3) EPC

(43) Date of publication:
**28.02.2007 Bulletin 2007/09**

(51) Int Cl.:
*A61K 47/32* (2006.01)   *A61K 9/70* (2006.01)

(21) Application number: **05720823.3**

(22) Date of filing: **15.03.2005**

(86) International application number:
**PCT/JP2005/004568**

(87) International publication number:
**WO 2005/097198 (20.10.2005 Gazette 2005/42)**

(84) Designated Contracting States:
**FR GB IT**

(30) Priority: **31.03.2004 JP 2004106842**

(71) Applicant: **Lintec Corporation**
**Tokyo 173-0001 (JP)**

(72) Inventor: **NOGAMI, Eiji**
**Saitama-shi,**
**Saitama 3360026 (JP)**

(74) Representative: **Benson, John Everett**
**J. A. Kemp & Co.,**
**14 South Square,**
**Gray's Inn**
**London WC1R 5JJ (GB)**

(54) **PREPARATION FOR ORAL ADMINISTRATION**

(57)    An object of the present invention is to provide an orally administered pharmaceutical composition comprising a drug-containing layer and a functional layer wherein cracks and gaps in the drug-containing layer are prevented even if the amount of glycerin contained in the drug-containing layer is not sufficient to cause bleeding of the drug-containing layer at the edges of the orally administered pharmaceutical composition, and to achieve the object, polyvinylpyrrolidone with a K value of 70 or more is contained as a base in the drug-containing layer.

EP 1 757 308 A1

**Description**

TECHNICAL FIELD

The present invention relates to an orally administered pharmaceutical composition.

BACKGROUND ART

Compliance may be diminished in the case of an orally administered pharmaceutical composition if it is rejected because the drug is bitter, astringent or otherwise unpleasant or if it causes nausea or vomiting.

For example, a solid preparation (such as a tablet or capsule), which is the normal form of an orally administered pharmaceutical composition, is difficult to swallow as is and must normally be taken with a large amount of water, which may detract from compliance. Elderly patients and infants in particular may be unable to swallow solid preparations, and compliance is often adversely affected. There is also the risk that a solid preparation may lodge in the trachea or adhere to the esophagus, leading to the formation of an esophageal tumor.

Orally administered pharmaceutical compositions have therefore been developed comprising a drug-containing layer and a functional layer (a layer having a particular function). An example of this is an orally administered pharmaceutical composition comprising a drug-containing layer and a water-swellable gel-forming layer, in which the water-swellable gel-forming layer is in the outermost layer of the orally administered pharmaceutical composition (International Patent Publication WO 02/087622).

DISCLOSURE OF THE INVENTION

In the process of producing an orally administered pharmaceutical composition comprising a drug-containing layer and a functional layer, once the drug-containing layer and functional layer have been layered together a step is required to work (stamp, for example) the layered product into a form, size and the like suited to oral administration. Since cracks and gaps may occur in the drug-containing layer during the process of working the layered product, a plasticizer such as glycerin, propylene glycol or glycerin triacetate is added to the drug-containing layer to give it an appropriate degree of plasticity and prevent cracks and gaps in the drug-containing layer.

Since cracks and gaps in the drug-containing layer cannot be adequately prevented if the amount of plasticizer added to the drug-containing layer is inadequate (normally 25% or less by weight of the drug-containing layer), an adequate amount (normally more than 25% by weight of the drug-containing layer) of plasticizer is added to the drug-containing layer. However, when an adequate amount of plasticizer is added to the drug-containing layer, the drug-containing layer may bleed from the edges of the orally administered pharmaceutical composition. Conversely, if the plasticizer is added in an amount so as not to cause bleeding of the drug-containing layer from the edges of the orally administered pharmaceutical composition, it will be impossible to prevent cracks and gaps in the drug-containing layer.

It is therefore an object of the present invention to provide an orally administered pharmaceutical composition comprising a drug-containing layer and a functional layer, wherein cracks and gaps in the drug-containing layer can be prevented even if the amount of plasticizer contained in the drug-containing layer is such as to not cause bleeding of the drug-containing layer from the edges of the orally administered pharmaceutical composition.

To solve these issues, the present invention provides an orally administered pharmaceutical composition comprising a drug-containing layer, wherein the drug-containing layer contains polyvinylpyrrolidone with a K value of 70 or more as a base.

When the drug-containing layer contains polyvinylpyrrolidone with a K value of 70 or more as a base, cracks and gaps in the drug-containing layer can be prevented even if the amount of plasticizer contained in the drug-containing layer is such as to not cause bleeding of the drug-containing layer from the edges of the orally administered pharmaceutical composition.

In the orally administered pharmaceutical composition of the present invention, the orally administered pharmaceutical composition is preferably a film preparation.

Cracks and gaps are likely to occur in the drug-containing layer when the orally administered pharmaceutical composition of the present invention is a film preparation, but if the drug-containing layer contains polyvinylpyrrolidone with a K value of 70 or more as a base, cracks and gaps in the drug-containing layer can be prevented even if the amount of plasticizer contained in the drug-containing layer is not sufficient to cause bleeding of the drug-containing layer from the edges of the orally administered pharmaceutical composition.

Moreover, when the orally administered pharmaceutical composition of the present invention is a film preparation the moisture content of the preparation can be minimized, thereby making the drug more stable (particularly in the case of an easily hydrolysable drug) than in a gelatinous preparation with a high moisture content. In this case the preparation is also easier to handle, and packaging costs are reduced.

Moreover, when the orally administered pharmaceutical composition of the present invention is a film preparation, even if the film strength of the drug-containing layer decreases when the amount of drug in the drug containing-layer is increased, the strength of the film preparation as a whole is maintained because of the film formability imparted to the functional layer. Consequently, the drug-containing layer may contain a wide variety of drugs in tiny to large quantities, as well as insoluble and bulky drugs that are likely to detract from film strength.

In the orally administered pharmaceutical composition of the present invention, the amount of the polyvinylpyrrolidone contained in the drug-containing layer is preferably 30% or more by weight of the drug-containing layer, and the amount of plasticizer contained in the drug-containing layer is preferably 2 to 25% by weight of the drug-containing layer.

When the amounts of plasticizer and polyvinylpyrrolidone with a K value of 70 or more contained in the drug-containing layer are within these ranges, cracks and gaps in the drug-containing layer can be effectively prevented, and bleeding of the drug-containing layer at the edges of the orally administered preparation can also be effectively prevented. When the drug-containing layer contains a plasticizer, the upper limit of the amount of polyvinylpyrrolidone with a K value of 70 or more contained in the drug-containing layer is 100% by weight minus the minimum amounts of drug and plasticizer contained in the drug-containing layer.

In the orally administered pharmaceutical composition of the present invention, the functional layer is preferably a water-swellable gel-forming layer, and the water-swellable gel-forming layer is preferably provided in the outermost layer of the orally administered pharmaceutical composition.

"Outermost layer" here means a layer that constitutes the outer surface of the orally administered pharmaceutical composition when the orally administered pharmaceutical composition is in the mouth of a patient, etc. Consequently, the "outermost layer" may be of course a layer that constitutes the outer surface of the orally administered pharmaceutical composition before administration, or may be a layer that does not constitute the outer surface of the orally administered pharmaceutical composition before administration, but that constitutes the outer surface of the orally administered pharmaceutical composition when it is in the patient's mouth. For example, even if an additional layer is provided as an outer layer outside the water-swellable gel-forming layer, if this outer layer is broken down or dissolved by saliva or other moisture inside the patient's mouth, the water-swellable gel-forming layer will constitute the outer surface of the orally administered pharmaceutical composition inside the patient's mouth, so that the water-swellable gel-forming layer becomes the outermost layer of the orally administered pharmaceutical composition.

The water-swellable gel-forming layer in the outermost layer of the orally administered pharmaceutical composition swells from saliva or other moisture inside the patient's mouth to form a gel, so that the orally administered pharmaceutical composition changes to a form of an easily-swallowable size, shape, elasticity, viscosity and the like. This makes it easy for the patient to take the orally administered pharmaceutical composition. Since there is also less risk that the orally administered pharmaceutical composition will lodge in the patient's trachea, it can be given safely even to elderly patients and infants. When the water-swellable gel-forming layer cannot gel adequately because the patient does not have enough saliva, the same effects can be obtained by administering the pharmaceutical composition together with a small amount of water or soaking it in water before administration. Much less water is required in this case than is required for administering a tablet, capsule or other solid preparation.

The water-swellable gel-forming layer in the outermost layer of the orally administered pharmaceutical composition can also mask the flavor (such as bitterness or astringency) and odor of the drug contained in the drug-containing layer when it swells from saliva or other moisture in the patient's mouth to form a gel.

In the orally administered pharmaceutical composition of the present invention, the functional layer is preferably a water-swellable gel-forming layer, and the water-swellable gel forming layer is preferably layered directly on the drug-containing layer.

When the water-swellable gel-forming layer is layered directly on the drug-containing layer, the polyvinylpyrrolidone contained in the drug-containing layer interacts with the water-swellable gel-forming agent contained in the water-swellable gel-forming layer. The greater the K value of the polyvinylpyrrolidone contained in the drug-containing layer, the greater the effects of this interaction, and the stronger the resulting gel. That is, when the water-swellable gel-forming layer is layered directly on the drug-containing layer, a gel is formed which is stronger than when the water-swellable gel-forming layer gels independently. Since in the orally administered pharmaceutical composition of the present invention the K value of the polyvinylpyrrolidone contained in the drug-containing layer is 70 or more, a gel is formed with sufficient gel strength, thereby improving the masking effect of the gel on the drug's flavor, odor and the like. The orally administered pharmaceutical composition also becomes easier to swallow, increasing the ease and safety of administration. These effects are even greater when the K value of the polyvinylpyrrolidone contained in the drug-containing layer is 90 or more.

In the orally administered pharmaceutical composition of the present invention, the water-swellable gel-forming agent contained in the water-swellable gel-forming layer is preferably a crosslinked polyacrylic acid.

When the water-swellable gel-forming agent contained in the water-swellable gel-forming layer is a crosslinked polyacrylic acid, gel strength is increased as discussed above due to combination with polyvinylpyrrolidone with a K value of 70 or more, and the masking effects of the gel on the flavor, odor and the like of the drug are further enhanced. The orally administered pharmaceutical composition also becomes easier to swallow, increasing the ease and safety of administration.

The present invention provides an orally administered pharmaceutical composition comprising a drug-containing layer and a function layer, wherein cracks and gaps in the drug-containing layer can be prevented even if the amount of plasticizer contained in the drug-containing layer is such as not to cause bleeding of the drug-containing layer at the edges of the orally administered pharmaceutical composition.

BRIEF DESCRIPTION OF THE DRAWINGS

Figure 1(a) is plane view showing one embodiment of the orally administered pharmaceutical composition of the present invention, while Figure 1(b) is a cross-section of the same embodiment.
Figure 2 is a cross-section showing another embodiment of the present invention.
Figure 3 is a cross-section showing the orally administered pharmaceutical composition of Figure 2 as it is administered.
Figure 4 is a cross-section showing another embodiment of the orally administered pharmaceutical composition of the present invention.
Figure 5 is a cross-section showing another embodiment of the orally administered pharmaceutical composition of the present invention.
Figure 6 is a cross-section showing another embodiment of the orally administered pharmaceutical composition of the present invention.
Figure 7 is a cross-section showing another embodiment of the orally administered pharmaceutical composition of the present invention.

BEST MODE FOR CARRYING OUT THE INVENTION

The present invention is explained in detail below.

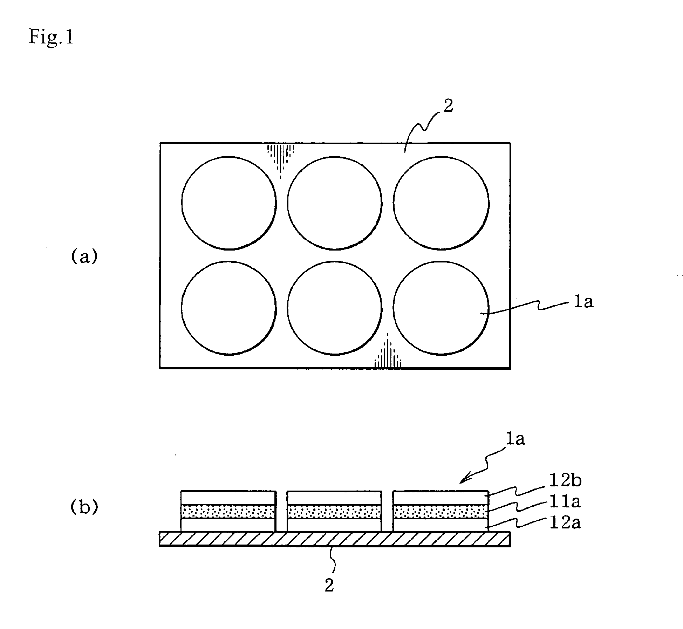

One embodiment of the orally administered pharmaceutical composition of the present invention is shown in Figure 1.

As shown in Figures 1(a) and (b), orally administered pharmaceutical composition 1a of this embodiment comprises drug-containing layer 11a, water-swellable gel-forming layer 12a layered directly on the underside of drug-containing layer 11a, and water-swellable gel-forming layer 12b layered directly on the top of drug-containing layer 11a, and is held on support base 2.

Orally administered pharmaceutical composition 1a is preferably a film preparation (sheet preparation). When orally administered pharmaceutical composition 1a is a film preparation, the stability of the drug contained in drug-containing layer 11a (particularly in the case of an easily hydrolysable drug) is greater than in a gelatinous preparation with a large moisture content because the moisture content in the preparation is minimized. The preparation is also easier to handle, and packaging costs are reduced.

Drug-containing layer 11a is the layer containing the drug to be administered.

The thickness of drug-containing layer 11a can be adjusted appropriately within the range that allows oral administration. When orally administered pharmaceutical composition 1a is a film preparation, the thickness of drug-containing layer 11a is preferably 0.1 to 1000 $\mu$m or more preferably 10 to 200 $\mu$m. If the thickness of drug-containing layer 11a is under 0.1 $\mu$m it will be hard to form the film precisely (that is, the drug content of drug-containing layer 11a will vary), while if the thickness is over 1000 $\mu$m the film will be stiff and harder to administer.

Drug-containing layer 11a contains polyvinylpyrrolidone with a K value of 70 or more as a base for maintaining the drug to be administered in the desired state in drug-containing layer 11a. In this way, cracks and gaps in drug-containing layer 11a can be prevented even if the amount of plasticizer contained in drug-containing layer 11a (normally 25% or less by weight of drug-containing layer 11a) is such as to not produce bleeding of drug-containing layer 11a at the edges of orally administered pharmaceutical composition 1a. Cracks and gaps are likely to occur in drug-containing layer 11a when orally administered pharmaceutical composition 1a is a film preparation, but gaps and cracks in drug-containing layer 11a can be prevented even in this case.

The polyvinylpyrrolidone with a K value of 70 or more serves as an excipient, stabilizer, binder and the like.

The K value of the polyvinylpyrrolidone contained in drug-containing layer 11a is preferably 70 or more and more preferably 80 or more.

The K value is the intrinsic viscosity of a polymer solution, and is also called the Fikentscher K value. The K value can be derived as the K value in the following formula:

$$\ln\eta_r = \{75\ K^2/(1 + 1.5\ KC) + K\} \times C$$

(wherein $\eta_r$ represents relative viscosity and C represents solute concentration (w/v%)).

Drug-containing layer 11a may contain only polyvinylpyrrolidone with a K value of 70 or more as a base, or may also contain another base. Examples of such other bases include crystal cellulose, carboxymethyl cellulose, hydroxypropyl cellulose, hydroxypropyl methyl cellulose, methyl cellulose, ethyl cellulose, cellulose acetate, cellulose acetate phthalate, hydroxypropyl methylcellulose phthalate, hydroxypropyl methylcellulose acetate succinate, carboxymethyl ethyl cellulose and other celluloses and derivatives thereof and pharmacologically acceptable salts (such as sodium salts) of these; alpha starch, oxidized starch, carboxymethyl starch sodium, hydroxypropyl starch, dextrin, dextran and other starches and derivatives thereof; sucrose, maltose, lactose, glucose, fructose, pullulan, xanthan gum, cyclodextrin and other sugars; xylitol, mannitol, sorbitol and other sugar alcohols; dimethylaminoethyl methacrylate-methacrylic acid copolymer, methacrylic acid-ethyl acrylate copolymer, methacrylic acid-methyl methacrylate copolymer, ethyl methacrylate-ammonium trimethyl chloride methacrylate copolymer, dimethylaminoethyl methacrylate-methyl methacrylate chloride copolymer, methacrylic acid-ethyl acrylate chloride copolymer and other acrylic acid derivatives; shellac; polyvinyl acetal diethyl aminoacetate; polyvinyl acetate; polyvinyl alcohol; polyvinylpyrrolidone with a K value under 70; vinyl acetate-vinylpyrrolidone copolymer; gum arabic, tragacanth gum and other natural gums; chitin, chitosan and other polyglycosamines; gelatin, casein, soy protein and other proteins; titanium oxide; calcium hydrogenphosphate; calcium carbonate; talc; stearic acid salts; magnesium aluminate metasilicate; magnesium silicate; anhydrous silicic acid and the like, and one or two or more of these may be added and used depending on the object.

Hydroxypropyl cellulose or hydroxypropyl methylcellulose phthalate is preferably used as the other base. These are useful when drug-containing layer 11a is in film form because they have excellent film-forming properties.

The amount of polyvinylpyrrolidone with a K value of 70 or more contained in drug-containing layer 11a can be adjusted appropriately according to the amount of the other base, etc., but is normally at least 30% by weight or preferably at least 35% by weight or more preferably at least 40% by weight of drug-containing layer 11a. If the content of polyvinylpyrrolidone with a K value of 70 or more is less than 30% by weight, cracks and gaps in drug-containing layer 11a cannot be prevented except by including in drug-containing layer 11a an amount of plasticizer (normally more than 25% by weight of drug-containing layer 11a) sufficient to cause bleeding of drug-containing layer 11a at the edges of orally administered pharmaceutical composition 1a.

The total content of the bases contained in drug-containing layer 11a is an amount sufficient to form drug-containing layer 11a, and can be adjusted appropriately according to the amount and types of bases and the like, but is normally at least 30% or preferably at least 60% or more preferably at least 70% by weight of drug-containing layer 11a. If the total content of the bases is less than 30%, drug-containing layer 11a will not form properly. The upper limit on the total content of bases is 100% by weight minus the minimum content of the drug contained in drug-containing layer 11a (or 100% by weight minus the minimum contents of the drug and plasticizer contained in drug-containing layer 11a if drug-containing layer 11a contains a plasticizer), and can be set appropriately depending on the type of drug, plasticizer and the like. For example, if the minimum content of the drug is 0.01% by weight of drug-containing layer 11a, the upper limit on the total content of bases is 99.99% by weight of drug-containing layer 11a. If the minimum contents of the drug and plasticizer are 0.01% and 2% by weight, respectively, of drug-containing layer 11a, the upper limit on the total content of bases is 97.99% by weight of drug-containing layer 11a.

Drug-containing layer 11a may contain a plasticizer to give drug-containing layer 11a suitable flexibility and make it easier to work, and to prevent cracks and gaps in drug-containing layer 11a. Examples of plasticizers include glycerin, propylene glycol, glycerin triacetate, polyethylene glycol, sorbitol, diethyl phthalate, triethyl citrate, lauric acid, sucrose and the like., and one or two of these can be selected and used. The amount of plasticizer contained in drug-containing layer 11a can be adjusted appropriately depending on the amount of polyvinylpyrrolidone with a K value of 70 or more contained in drug-containing layer 11a and the like, but is normally 2 to 25% or preferably 4 to 21% or more preferably 6 to 17% by weight of drug-containing layer 11a. If the plasticizer content exceeds 25% by weight drug-containing layer 11a will bleed from the edges of orally administered pharmaceutical composition 1a, while if it is less than 2% by weight the plasticizer will not function effectively as such.

When drug-containing layer 11a contains a plasticizer, if the amount of polyvinylpyrrolidone with a K value of 70 or more contained in drug-containing layer 11a is 30% by weight or more of drug-containing layer 11a and the amount of plasticizer contained in drug-containing layer 11a is 2 to 25% by weight of drug-containing layer 11a, cracks and gaps in drug-containing layer 11a can be effectively prevented, as can bleeding of drug-containing layer 11a from the edges of orally administered pharmaceutical composition 1a.

The drug contained in drug-containing layer 11a is not particularly limited as long as it is a drug to be administered to a patient, and one that can be orally administered. Examples of drugs that can be orally administered include for example drugs that affect the central nervous system, such as amobarbital, estazolam, triazolam, nitrazepam, pentobarbital and other sleeping drugs, amitriptyline hydrochloride, imipramine hydrochloride, oxazolam, chlordiazepoxide, chlorpromazine, diazepam, sulpiride, haloperidol and other psychotropics, trihexyphenidyl, levodopa and other antiparkinsonians, aspirin, isopropylantipyrine, indomethacin, diclofenac sodium, mefenamic acid, streptokinase, streptodornase, serrapeptase, pronase and other analgesics and anti-inflammatories, and ATP, vinpocetine and other central nervous metabolism enhancers; drugs that affect the respiratory system, such as carbocysteine, bromhexine hydrochloride and other expectorants, and azelastine hydrochloride, oxatomide, theophylline, terbutaline sulfate, tranilast, procaterol hydrochloride, ketotifen fumarate and other antiasthmatics; drugs that affect the circulatory system, such as aminophylline, digitoxin, digoxin and other cardiac stimulants, ajmaline, disopyramide, procainamide hydrochloride, mexiletine hydrochloride and other antiarrhythmics, amyl nitrite, alprenolol hydrochloride, isosorbide dinitrate, nicorandil, oxyfedrine, dipyridamole, dilazep hydrochloride, diltiazem hydrochloride, nitroglycerine, nifedipine, verapamil hydrochloride and other anti-angina drugs, kallidinogenase and other peripheral vasodilators, atenolol, captopril, clonidine hydrochloride, metoprolol tartrate, spironolactone, triamterene, trichlormethiazide, nicardipine, hydralazine hydrochloride, hydrochlorothiazide, prazosin hydrochloride, furosemide, propranolol hydrochloride, enalapril maleate, methyldopa, labetalol hydrochloride, reserpine and other anti-hypertensives, and clofibrate, dextran sulfuric acid, nicomol, niceritrol and other anti-arteriosclerotics; blood and hematopoietic drugs, such as carbazochrome sodium sulfate, tranexamic acid and other hemostatics, ticlopidine hydrochloride, warfarin potassium and other anti-thrombosis drugs, and iron sulfate and other anemia treatment drugs; drugs that affect the digestive tract, such as azulene, aldioxa, cimetidine, ranitidine hydrochloride, famotidine, teprenone, rebamipide and other anti-ulcer drugs, domperidone, metoclopramide and other antiemetics, sennoside and other cathartics, digestive enzyme regulators, and glycyrrhizin, liver extract preparations and other drugs for treatment of liver disease; drugs that affect metabolic conditions, such as glibenclamide, chlorpropamide, tolbutamide and other anti-diabetic drugs, and allopurinol, colchicine and other gout treatment drugs; ophthalmological drugs such as acetazolamide; otorhinological drugs, such as diphenidol hydrochloride, betahistine mesylate and other anti-vertigo drugs; chemotherapy drugs and antibiotics, such as isoniazid, ethambutol hydrochloride, ofloxacin, erythromycin stearate, cefaclor, norfloxacin, fosfomycin calcium, minocycline hydrochloride, rifampicin and rokitamycin; anti-malignant tumor drugs, such as cyclophosphamide and tegafur; immunosuppressors such as azathioprine; hormones and endocrine treatment drugs such as luteal hormone, salivary gland hormone, thiamazole, prednisolone, betamethasone, liothyronine and levothyroxine; autacoids such as clemastine fumarate, D-chlorpheniramine maleate and other antihistamines; and alfacalcidol, cobamamide, tocopherol nicotinate, mecobalamin and other vitamins and the like, and one or two or more of these can be selected and used according to the therapeutic or prophylactic objective or the like.

The amount of drug contained in drug-containing layer 11a is not particularly limited and may be determined appropriately according to the type of drug, but is normally 70% or less or preferably 40% or less or more preferably 30% or less by weight of drug-containing layer 11a. If the drug content exceeds 70% by weight, film strength will be adversely affected when orally administered pharmaceutical composition 1a is a film preparation. The lower limit on the drug content is set appropriately according to the type of drug contained in drug-containing layer 11a but is normally about 0.01% by weight.

A wide range of drugs that are administered in tiny to large doses may be contained in drug-containing layer 11a. A tiny dose here means 1 mg or less per administration, while a large dose means 300 mg or more per administration.

When orally administered pharmaceutical composition 1a is a film preparation, drug-containing layer 11a may still contain a wide range of drugs that are administered in tiny to large doses, as well as insoluble and bulky drugs that are likely to detract from film strength. This is because drug-containing layer 11a and water-swellable gel-forming layers 12a and 12b are formed as separate layers, so that even if the film strength of drug-containing layer 11a declines when the amount of drug in drug-containing layer 11a is increased, the strength of the film preparation as a whole is maintained because of the film formability conferred on water-swellable gel-forming layers 12a and 12b.

Water-swellable gel-forming layers 12a and 12b are layers that contain a water-swellable gel-forming agent and can swell from moisture to form a gel.

The thickness of water-swellable gel-forming layers 12a and 12b can be set appropriately within a range that allows oral administration, but is preferably 10 to 1000μ m or more preferably 20 to 500 μm when orally administered pharmaceutical composition 1a is a film preparation. If the water-swellable gel-forming layers 12a and 12b is less than 10 μm thick the gel will not form properly, and the ability of the water-swellable gel-forming layers 12a and 12b to mask the flavor and/or smell of the drug will be inadequate, while if the water-swellable gel-forming layers 12a and 12b is over 1000 μm thick it will be difficult to ingest because it will not swell adequately to form a gel simply from the saliva inside the patient's mouth.

The water-swellable gel-forming agent contained in water-swellable gel-forming layers 12a and 12b is not particularly limited as to type as long as it can swell from moisture to form a gel, and it may or may not be crosslinked.

Examples of water-swellable gel-forming agents include carboxyvinyl polymers, starch and derivatives thereof, agar, alginic acid, arabinogalactan, galactomannan, cellulose and derivatives thereof, carrageen, dextran, tragacanth, gelatin, pectin, hyaluronic acid, gellan gum, collagen, casein, xanthan gum and the like, and one or two or more of these can be selected and used.

The water-swellable gel-forming agent contained in water-swellable gel-forming layers 12a and 12b is preferably a crosslinked carboxyvinyl polymer, particularly a crosslinked polyacrylic acid. A crosslinked polyacrylic acids does not adversely affect the film-forming capabilities of a film-forming agent, and exhibits good gel strength when swollen. Moreover, a crosslinked polyacrylic acid can interact during gel formation with the polyvinylpyrrolidone with a K value of 70 or more contained in drug-containing layer 11a to form a gel with good gel strength.

Crosslinking can be accomplished with a crosslinking agent suited to the type of molecule to be crosslinked. Crosslinking agents for polyacrylic acids include for example polyvalent metal compounds. Specific examples of such polyvalent metal compounds include calcium chloride, magnesium chloride, aluminum chloride, aluminum sulfate, potassium alum, iron alum chloride, ammonium alum, ferric sulfate, aluminum hydroxide, aluminum silicate, aluminum phosphate, iron citrate, magnesium oxide, calcium oxide, zinc oxide, zinc sulfate and the like. Using a trivalent metal compound increases the degree of crosslinking of the polyacrylic acid, and allows a strong gel to be formed when the crosslinked polyacrylic acid interacts with the polyvinylpyrrolidone with a K value of 70 or more.

The amount of the water-swellable gel-forming agent contained in water-swellable gel-forming layers 12a and 12b can be adjusted appropriately according to the type of water-swellable gel-forming agent and the like, but is preferably 15 to 70% by weight of the water-swellable gel forming layer.

When orally administered pharmaceutical composition 1a is a film preparation, water-swellable gel-forming layers 12a and 12b need to be made in film form, and in this case it is desirable to include a film-forming agent in water-swellable gel-forming layers 12a and 12b in order to improve the film-formability of water-swellable gel-forming layers 12a and 12b.

The film forming agent is not particularly limited as to type as long as it has film-forming ability. Specific examples of film-forming agents include polyvinyl alcohol, polyvinylpyrrolidone, polyvinyl acetate, polyvinyl acetate phthalate, hydroxyalkyl cellulose (such as hydroxypropyl cellulose, hydroxypropyl methyl cellulose, hydroxymethyl cellulose and hydroxyethyl cellulose), alkyl cellulose (such as methyl cellulose and ethyl cellulose), carboxyalkyl cellulose (such as carboxymethyl cellulose), (meth)acrylic acid and esters thereof, xanthan gum, carrageenan, alginic acid and the like.

The amount of the film-forming agent contained in water-swellable gel-forming layers 12a and 12b can be adjusted appropriately according to the type of film-forming agent and the like, but is preferably 30 to 85% by weight of water-swellable gel forming layers 12a and 12b.

The film-forming agent contained in water-swellable gel-forming layers 12a and 12b is preferably water-soluble. When the film-forming agent is water-soluble, moisture penetrates water-swellable gel-forming layers 12a and 12b more easily, promoting swelling and gel formation of water-swellable gel-forming layers 12a and 12b in the mouth.

Examples of water-soluble film-forming agents including polyvinyl alcohol, hydroxyalkyl cellulose such as hydroxypropyl cellulose, hydroxypropyl methyl cellulose and methyl cellulose, and polyvinylpyrrolidone, xanthan gum, carrageenan, alginic acid and the like.

A plasticizer may be contained in water-swellable gel-forming layers 12a and 12b in order to confer a suitable degree of flexibility to the water-swellable gel-forming layers. Examples of plasticizers include propylene glycol, polyethylene glycol, glycerin, sorbitol, glycerin triacetate, diethyl phthalate, triethyl citrate, lauric acid, sucrose and the like. one or two of these may be selected and used.

A masking agent may be contained in water-swellable gel-forming layers 12a and 12b in order to mask the flavor and odor of the drug contained in the drug-containing layer 11a. Including a masking agent in water-swellable gel-forming layers 12a and 12b serves to improve the ability of water-swellable gel-forming layers 12a and 12b to mask the flavor and odor of the drug. Examples of masking agents include those that contribute acidity such as citric acid, tartaric acid and fumaric acid, sweeteners such as saccharine, glycyrrhizic acid, sucrose, fructose and mannitol, cooling agents such as menthol, mint oil, peppermint and spearmint, and natural and artificial aromatics and the like, and one or two of these can be selected and used.

When polyvinyl alcohol and the like are included as film-forming agents in water-swellable gel-forming layers 12a and 12b, these film-forming agents may also serve as masking agents. It is desirable to use a film-forming agent having such a masking effect, and it is similarly desirable to use a water-swellable gel-forming agent having such a masking effect.

Preservatives such as methyl- and propyl-hydroxybenzoate and colorants such as edible lake colorants can also be contained in water-swellable gel-forming layers 12a and 12b.

In general, mixing of these additives into water-swellable gel-forming layers 12a and 12b weakens water-swellable gel-forming layers 12a and 12b, making it easier for moisture to penetrate water-swellable gel-forming layers 12a and 12b, so that the water-swellable gel-forming agent swells and forms a gel more easily due to moisture penetrating water-swellable gel-forming layers 12a and 12b.

As shown in Figures 1(a) and (b), water-swellable gel-forming layers 12a and 12b are layered, respectively, on the bottom and top of drug-containing layer 11a in orally administered pharmaceutical composition 1a. Consequently, when orally administered pharmaceutical composition 1a is administered in a patient's mouth, water-swellable gel-forming layers 12a and 12b swell from saliva or other moisture to form a gel, so that drug-containing layer 11a becomes covered with gel, masking the flavor, odor and the like of the drug contained in drug-containing layer 11a. In particularly, as shown in Figures 1(a) and (b), since water-swellable gel-forming layers 12a and 12b are layered over the entire bottom and top, respectively, of drug-containing layer 11a, virtually all of drug-containing layer 11a becomes covered with gel, effectively masking the flavor, odor and the like of the drug contained in drug-containing layer 11a.

As shown in Figures 1(a) and (b), water-swellable gel-forming layers 12a and 12b are in the outermost layer of orally administered pharmaceutical composition 1a. Consequently, when water-swellable gel-forming layers 12a and 12b gel, orally administered pharmaceutical composition 1a changes to an easily swallowed size, shape, elasticity, viscosity and the like. This makes it easy for a patient to take orally administered pharmaceutical composition 1a. Since there is less risk that orally administered pharmaceutical composition 1a will lodge in the patient's trachea, moreover, it can be safely given even to elderly patients and infants. In the case of patients who do not have enough saliva to adequately gel water-swellable gel-forming layers 12a and 12b, the same effects can be obtained by administering the pharmaceutical composition together with a small amount of water or by soaking it in water before administration. Much less water is required in this case than is required for administering a tablet, capsule or other solid preparation.

As shown in Figures 1(a) and (b), water-swellable gel-forming layers 12a and 12b are layered directly on drug-containing layer 11a in orally administered pharmaceutical composition 1a. When orally administered pharmaceutical composition 1a is constituted in this way, the strength of the gel formed by gelling of water-swellable gel-forming layers 12a and 12b (1) is much greater when the K value of the polyvinylpyrrolidone contained in drug-containing layer 11a is 70 or more or preferably 90 or more, (2) is much greater when the crosslinking agent for the polyacrylic acid contained in water-swellable gel-forming layers 12a and 12b is a trivalent metal compound than when it is a bivalent metal compound, and (3) is much greater than that of water-swellable gel-forming layers 12a and 12b by themselves due to the interaction of the polyvinylpyrrolidone contained in drug-containing layer 11a with the water-swellable gel-forming agent contained in water-swellable gel-forming layers 12a and 12b. This increase in gel strength not only enhances the masking effect of the gel on the flavor, odor and the like of the drug, but also makes orally administered pharmaceutical composition 1a easier to swallow, further enhancing the ease and safety of administration.

Orally administered pharmaceutical composition 1a may be produced by the following first method and second method.

[First Method]

A suspension obtained by adding a water-swellable gel-forming agent and a film-forming agent (with purified water for example as the solvent) is painted, sprayed or the like on the top of support base 2, which is a plastic film, mount or the like, and dried to form water-swellable gel-forming layer 12b. Next, a suspension obtained by adding a drug and additives such as excipients, binders, disintegrators and the like (with ethanol for example as the solvent) is painted, sprayed or the like on the top of water-swellable gel-forming layer 12b, and dried to form drug-containing layer 11a. Next, a suspension obtained by adding a water-swellable gel-forming agent and a film-forming agent is painted, sprayed or the like on the top of drug-containing layer 11a, and dried to form water-swellable gel-forming layer 12a. A layered body is thus produced comprising water-swellable gel-forming layer 12b, drug-containing layer 11a and water-swellable drug-containing layer 12a layered in that order, and this layered body can be punched out in a circular shape to produce orally administered pharmaceutical composition 1a. In this case, cracks and gaps do not occur in drug-containing layer 11a even if the amount of glycerin contained in drug-containing layer 11a is not sufficient to cause bleeding of drug-containing layer 11a at the edges of orally administered pharmaceutical composition 1a. In this embodiment the layered body was punched out in a circular shape, but it could also be punched out in an oval, polygonal or any other shape.

[Second method]

An intermediate is produced comprising water-swellable gel-forming layer 12b and drug-containing layer 11a layered in that order by first forming water-swellable gel-forming layer 12b on the top of support base 2, which is a plastic film, mount or the like, and then forming drug-containing layer 11a on the top of water-swellable gel-forming layer 12b. Another intermediate is produced in the same way comprising water-swellable gel-forming layer 12a and drug-containing layer 11a layered in that order on the top of support base 2. The drug-containing layers 11a of the two intermediates are then heat fused to each other. In this way, a layered body is produced comprising water-swellable gel-forming layer 12b, drug-containing layer 11a and water-swellable gel-forming layer 12a layered in that order, and this layered body can be punched out in a circular shape to produce orally administered pharmaceutical composition 1a. In this case, cracks and gaps do not occur in drug-containing layer 11a even if the amount of glycerin contained in drug-containing layer 11a is not sufficient to produce bleeding of drug-containing layer 11a at the edges of orally administered pharmaceutical composition 1a. The two drug-containing layers 11a can be heat fused together because the polyvinylpyrrolidone with a K value of 70 or more contained in the drug-containing layers 11a of the two intermediates is a thermoplastic polymer. In this embodiment the layered body was punched out in a circular shape, but it could also be punched out in an oval, polygonal or any other shape.

The functional layer of orally administered pharmaceutical composition 1a is a water-swellable gel-forming layer, but the type of functional layer in the orally administered pharmaceutical composition of the present invention is not limited to a water-swellable gel-forming layer.

Examples of functional layers other than water-swellable gel-forming layers include adhesive layers provided between layers (such as between a drug-containing layer and a water-swellable gel-forming layer, between a drug-containing layer and a drug-containing layer, or between a water-swellable gel-forming layer or drug-containing layer and a support base).

The adhesive contained in an adhesive layer is not particularly limited as long as it is a pharmacologically acceptable adhesive, and examples of adhesives that exhibit adhesiveness when included in a solvent include carboxyvinyl polymers, sodium polyacrylate and other polyacrylic acids or pharmacologically acceptable nontoxic salts thereof, acrylic acid copolymers or pharmacologically acceptable salts thereof, carboxymethylcellulose, sodium salts and other hydrophilic cellulose derivatives, pullulan, povidone, karaya gum, pectin, xanthan gum, tragacanth, alginic acid, gum arabic, acidic polysaccharides and derivatives and pharmacologically acceptable salts thereof and the like, while adhesives that exhibit adhesiveness when heated (that is, heat-fusable adhesives) include for example vinyl acetate, polyvinylpyrrolidone and other homopolymers and copolymers of vinyl acetate and vinylpyrrolidone and the like.

Another example of a functional layer is a layer for adjusting film thickness. When the orally administered pharmaceutical composition of the present invention is a film preparation, handling of the orally administered pharmaceutical composition of the present invention can be improved by using such a layer to increase the film thickness.

In orally administered pharmaceutical composition 1a the water-swellable gel-forming layers are layered directly on the drug-containing layer, but the water-swellable gel-forming layers may also be layered on the drug-containing layer via another functional layer

(intermediate layer).

Orally administered pharmaceutical composition 1a has a water-swellable gel-forming layer on both the top and bottom sides of the drug-containing layer, but the number of functional layers is not particularly limited in the orally administered pharmaceutical composition of the present invention.

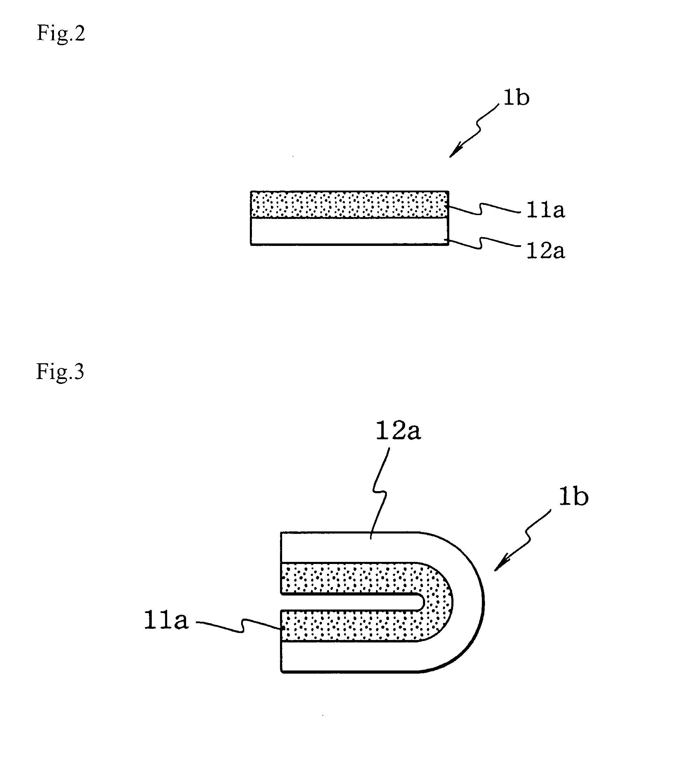

Figure 2 shows one embodiment in which the orally administered pharmaceutical composition of the present invention has one functional layer. As shown in Figure 2, orally administered pharmaceutical composition 1b has drug-containing layer 11a and water-swellable gel-forming layer 12a, which is layered directly on the underside of drug-containing layer 11a. When administering orally administered pharmaceutical composition 1b, as shown in Figure 3, orally administered pharmaceutical composition 1b is folded so that the top and bottom of drug-containing layer 11a are covered by water-swellable gel-forming layer 12a, thereby providing the same effects as orally administered pharmaceutical composition 1a in which water-swellable gel-forming layers 12a and 12a are layered, respectively, on the top and bottom of drug-containing layer 11a.

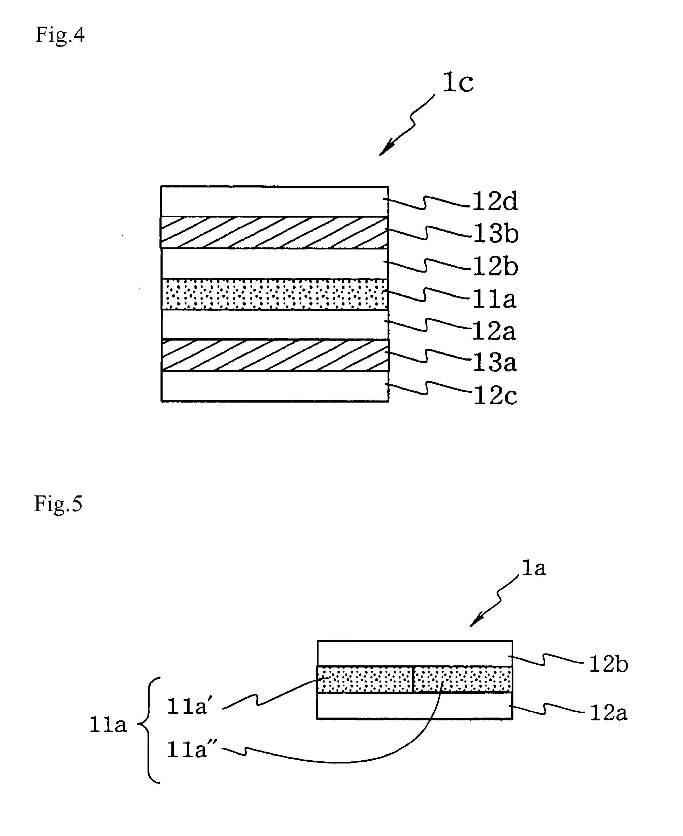

Figure 4 shows one embodiment in which the orally administered pharmaceutical composition of the present invention has multiple functional layers layered on the bottom of the drug-containing layer and multiple functional layers layered on the top of the drug-containing layer. As shown in Figure 4, orally administered pharmaceutical composition 1c has drug-containing layer 11a, water-swellable gel-forming layer 12a layered directly on the bottom of drug-containing layer 11a, water-swellable gel-forming layer 12c layered on the bottom of water-swellable gel-forming layer 12a with intermediate layer 13a in between, water-swellable gel-forming layer 12b layered directly on the top of drug-containing layer 11a, and water-swellable gel-forming layer 12d layered on the top of water-swellable gel-forming layer 12b with intermediate layer 13b in between. In orally administered pharmaceutical composition 1c, water-swellable gel-forming layers 12a and 12b layered directly on drug-containing layer 11a serve mainly to mask the flavor, odor and the like of the drug contained in drug-containing layer 11a, while water-swellable gel-forming layers 12c and 12d provided on the outside of orally administered pharmaceutical composition 1c serve mainly to make orally administered pharmaceutical composition 1c easier to swallow, contributing to ease and safety of administration.

In orally administered pharmaceutical composition 1a, as shown in Figure 5a, one drug-containing layer 11a may be formed by drug-containing layers 11a' and 11a", which are formed side by side.

Orally administered pharmaceutical composition 1a has one drug-containing layer, but the number of drug-containing layers is not particularly limited in the orally administered pharmaceutical composition of the present invention.

When the orally administered pharmaceutical composition of the present invention has multiple drug-containing layers, the drug-containing layers may be layered directly on one another or via intermediate layers.

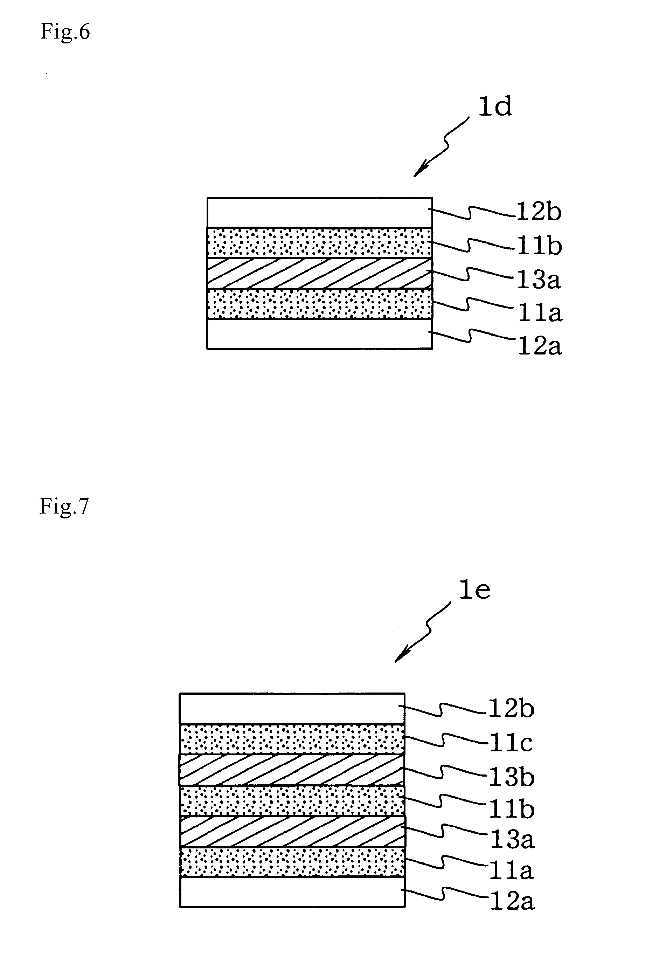

Figure 6 shows one embodiment in which the orally administered pharmaceutical composition of the present invention has two drug-containing layers. As shown in Figure 6, orally administered pharmaceutical composition 1d has two drug-containing layers 11a and 11b layered with intermediate layer 13a in between, as well as water-swellable gel-forming layer 12a layered directly on the underside of drug-containing layer 11a and water-swellable gel-forming layer 12b layered directly on the top of drug-containing layer 11b.

Figure 7 shows one embodiment in which the orally administered pharmaceutical composition of the present invention has three drug-containing layers. As shown in Figure 7, orally administered pharmaceutical composition 1e has three drug-containing layers 11a, 11b and 11c layered with the two intermediate layers 13a and 13b in between, as well as water-swellable gel-forming layer 12a layered directly on the underside of drug-containing layer 11a, which is the bottommost of the drug-containing layers, and water-swellable gel-forming layer 12b layered directly on the top of drug-containing layer 11c, which is the topmost of the drug-containing layers.

Orally administered pharmaceutical composition 1a is a layered pharmaceutical composition comprising multiple layers stacked flatly atop one another, but the orally administered pharmaceutical composition of the present invention may be in any form as long as it is layered, and may for example be flat or folded (see Figure 3).

Orally administered pharmaceutical composition 1a is supported by support base 2, but it may be peeled off of support base 2. Moreover, 6 individual orally administered pharmaceutical compositions 1a are shown supported on support base 2 (see Figure 1), but there is no particular limit on the number of orally administered pharmaceutical compositions 1a that can be supported on support base 2.

[Examples]

The present invention is explained in more detail below using examples and test examples.

[Production Example 1] Production of layered body

(1) Preparation of water-swellable gel-forming layer forming liquid (Coating Liquid A)

Coating liquid A was prepared with the composition shown in Table 1 below for purposes of forming a water-swellable gel-forming layer. 1 g of potassium alum was added to 140 g of purified water, and completely dissolved by agitation for about 10 minutes. Next, 6 g of polyacrylic acid (Carbopol 974P (BF Goodrich) was added gradually with agitation, and completely dissolved by being agitated for about 1 hour. Next, 17 g of polyvinyl alcohol (Gohsenol EG-05T, Nippon Gohsei) was added gradually to the solution with agitation, and completely dissolved by agitation with heating at 70°C for about 1 hour. 2 g of glycerin (Kanto Chemical Co., Inc.) was then added and agitated for about 5 minutes.

Polyacrylic acid is crosslinked by the aluminum ions produced by ionization of potassium alum, and the crosslinked polyacrylic acid serves as a water-swellable gel-forming agent, while the polyvinyl alcohol serves as a film-forming agent.

[Table 1]

|  | Recipe G-1 |
| --- | --- |
| Polyvinyl alcohol | 17 g |
| Polyacrylic acid | 6 g |
| Potassium alum | 1 g |
| Glycerin | 2 g |
| Purified water | 140 g |

(2) Formation of water-swellable gel-forming layer

Coating liquid A was thoroughly degassed and, using an applicator with the gaps aligned so as to achieve a dried coating volume of 30 g/m$^2$, spread on the non-release-treated side of a polyethylene terephthalate film (Lintec Corporation, SP-PET3801) which had been release treated on the opposite side with a silicone resin, and dried for 10 minutes at 80°C to form a water-swellable gel-forming layer.

(3) Preparation of drug-containing layer forming liquid

(Coating Liquid B)

Coating liquid B was prepared with the composition shown in Table 2 below for purposes of forming a drug-containing layer. 7 g of the stomach ulcer drug famotidine and 0.2 g of titanium oxide were added to 17 g or 50 g of ethanol and thoroughly dispersed with a homogenizer, after which 15 g of polyvinylpyrrolidone (PVP K-30 or PVP K-90, ISP Japan) and 3 g or 8 g of glycerin (Kanto Chemical Co., Inc.) were added gradually with agitation, and completely dissolved by being agitated for about 20 minutes. PVP K-30 has a K value of 30, while PVP-90 has a K value of 90

[Table 2]

|  | Recipe D-1 | Recipe D-2 | Recipe D-3 |
| --- | --- | --- | --- |
| Famotidine | 7 g | 7 g | 7 g |
| PVP K-30 | 15 g | 15 g |  |
| PVP K-90 |  |  | 15 g |
| Titanium oxide | 0.2 g | 0.2 g | 0.2 g |
| Glycerin | 3 g | 8 g | 3 g |
| Ethanol | 17 g | 17 g | 50 g |

(4) Formation of drug-containing layer

Coating liquid B was thoroughly degassed and, using an applicator with the gaps aligned so as to achieve a dried coating volume of 70 g/m$^2$, spread on the aforementioned water-swellable gel forming layer and dried for about 15 minutes at 80°C to form a drug-containing layer. When the drug-containing layer was formed using Recipe D-1, the amounts of famotidine, PVP-30 and glycerin contained in the drug containing-layer constituted about 28%, 60% and 12% by weight, respectively, of the drug-containing layer. When the drug-containing layer was formed using Recipe D-2, the amounts of famotidine, PVP-30 and glycerin in the drug-containing layer constituted about 23%, 50% and 26% by weight, respectively, of the drug-containing layer. When the drug-containing layer was formed using Recipe D-3, the amounts of famotidine, PVP-90 and glycerin in the drug containing layer constituted about 28%, 60% and 12%, respectively, of the drug-containing layer.

In this way an intermediate was produced comprising a water-swellable gel-forming layer and a drug-containing layer layered successively on the aforementioned polyethylene terephthalate film. Another intermediate was produced in the same way.

(5) Production of orally administered pharmaceutical composition by heat fusion.

Next, the drug-containing layers of the intermediates were heat fused together under conditions of 100°C, 1 kgf/cm$^2$, 2 seconds.

A layered body was thus produced comprising a water-swellable gel-forming layer, a drug-containing layer and a water-swellable gel-forming layer layered successively on the aforementioned polyethylene terephthalate film.

[Text Example 1]

A layered body (comparative product) was produced as in Production Example 1 using Recipe G-1 for Coating Liquid A and Recipe D-1 for Coating Liquid B. Another layered body (product of the invention) was also produced as in Production Example 1 using Recipe G-1 for Coating Liquid A and Recipe D-3 for Coating Liquid B. The layered bodies were punched out in cylindrical shapes 15 mm in diameter to produce orally administered pharmaceutical compositions which were then taken without water by 10 randomly selected test subjects, and the gelling ability and ability to mask the flavor of the drug were evaluated according to the following 5-level scale.

[Scale for evaluating gelling ability and masking ability]

1 Product of invention worse than comparative product
2 Product of invention somewhat worse than comparative product
3 Product of invention equal to comparative product
4 Product of invention somewhat better than comparative product
5 Product of invention better than comparative product

[Table 3]

| Evaluation | Test subject | | | | | | | | | | |
|---|---|---|---|---|---|---|---|---|---|---|---|
| | 1 | 2 | 3 | 4 | 5 | 6 | 7 | 8 | 9 | 10 | Mean |
| Gelling ability | 5 | 4 | 5 | 5 | 4 | 5 | 5 | 5 | 5 | 5 | 4.8 |
| Masking ability | 4 | 5 | 4 | 5 | 4 | 5 | 5 | 4 | 5 | 4 | 4.5 |

As shown in Table 3, the product of the invention was judged to be superior to the comparative product in terms of gelling ability and ability to mask the flavor of the drug.

[Test Example 2] Evaluation test for occurrence of cracks and gaps

A layered body (comparative product 1) was produced as in Production Example 1 using Recipe G-1 for Coating Liquid A and Recipe D-1 for Coating Liquid B. A layered body (comparative product 2) was also produced as in Production Example 1 using Recipe G-1 for Coating Liquid A and Recipe D-2 for Coating Liquid B. Another layered body (product of the invention) was produced as in Production Example 1 using Recipe G-1 for Coating Liquid A and Recipe D-3 for Coating Liquid B. Each layered body was punched 10 times, and the occurrence of cracks and gaps in the drug-containing layer was evaluated. As a result, the rate of occurrence of cracks and gaps was 40% in comparative product 1, 0% in comparative product 2 and 0% in the product of the invention.

[Test Example 3] Test to evaluate bleeding of drug-containing layer during storage

Comparative product 1, comparative product 2 and the product of the invention were produced as in Test Example 2, packaged in aluminum bags and stored for 1 month under dry conditions at 50°C, and the presence or absence of bleeding of the drug-containing layer was evaluated. As a result, the rate of occurrence of bleeding was 0% for comparative product 1, 100% for comparative product 2 and 0% for the product of the invention.

As shown by the results of Test Examples 2 and 3, bleeding of the drug-containing layer did not occur in the case of comparative product 1, in which the glycerin contained in the drug-containing layer was insufficient to cause bleeding of the drug-containing layer, but cracks and gaps did occur in the drug-containing layer. Cracks and gaps did not occur in the case of comparative product 2, in which the glycerin contained in the drug-containing layer was sufficient to cause bleeding of the drug-containing layer, but bleeding of the drug-containing layer did occur. By contrast, with the product of the present invention there were no cracks and gaps in the drug-containing layer and no bleeding of the drug-containing layer even though the drug-containing layer contained sufficient glycerin to cause bleeding.

INDUSTRIAL APPLICABILITY

The present invention provides an orally administered pharmaceutical composition comprising a drug-containing layer and a functional layer, wherein cracks and gaps in the drug-containing layer can be prevented even if the amount of a plasticizer contained in the drug-containing layer is not sufficient to cause bleeding of the drug-containing layer at the edges of the orally administered pharmaceutical composition.

Claims

1. An orally administered pharmaceutical composition comprising a drug-containing layer and a functional layer, wherein the drug-containing layer contains polyvinylpyrrolidone with a K value of 70 or more as a base.

2. The orally administered pharmaceutical composition according to Claim 1, wherein the orally administered pharmaceutical composition is a film preparation.

**EP 1 757 308 A1**

3. The orally administered pharmaceutical composition according to Claim 1 or 2, wherein the amount of the polyvinylpyrrolidone contained in the drug-containing layer is 30% by weight or more of the drug-containing layer, and the amount of a plasticizer contained in the drug-containing layer is 2 to 25% by weight of the drug-containing layer.

4. The orally administered pharmaceutical composition according to any of Claims 1 to 3, wherein the functional layer is a water-swellable gel-forming layer, and the water-swellable gel-forming layer is provided in the outermost layer of the orally administered pharmaceutical composition.

5. The orally administered pharmaceutical composition according to any of Claims 1 to 4, wherein the functional layer is a water-swellable gel-forming layer, and the water-swellable gel forming layer is layered directly on the drug-containing layer.

6. The orally administered pharmaceutical composition according to Claim 5, wherein a water-swellable gel-forming agent contained in the water-swellable gel-forming layer is a crosslinked polyacrylic acid.

13

Fig.1

(a)

(b)

Fig.2

1b

11a

12a

Fig.3

12a

1b

11a

Fig.4

1c

12d
13b
12b
11a
12a
13a
12c

Fig.5

1a

12b
11a'
11a
11a''
12a

Fig.6

1d
12b
11b
13a
11a
12a

Fig.7

1e
12b
11c
13b
11b
13a
11a
12a

## INTERNATIONAL SEARCH REPORT

| | International application No. |
| --- | --- |
| | PCT/JP2005/004568 |

| A. CLASSIFICATION OF SUBJECT MATTER |
| --- |
| Int.Cl⁷ A61K47/32, A61K9/70 |

According to International Patent Classification (IPC) or to both national classification and IPC

| B. FIELDS SEARCHED |
| --- |
| Minimum documentation searched (classification system followed by classification symbols) |
| Int.Cl⁷ A61K47/32, A61K9/70 |

Documentation searched other than minimum documentation to the extent that such documents are included in the fields searched

| | | | |
| --- | --- | --- | --- |
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)

| C. DOCUMENTS CONSIDERED TO BE RELEVANT |
| --- |

| Category* | Citation of document, with indication, where appropriate, of the relevant passages | Relevant to claim No. |
| --- | --- | --- |
| X<br>Y | JP 2001-28074 A  (Taiho Pharmaceutical<br>Co., Ltd.),<br>10 October, 2001 (10.10.01),<br>Full text; Claims; examples 1, 2, 4, 5<br>(Family: none) | 1-5<br>6 |
| Y | WO 02/87622 A1  (LINTEC CORP.),<br>07 November, 2002 (07.11.02),<br>Full text<br>& EP 1391212 A1        & AU 2002/255267 A1<br>& KR 2004/2391 A        & US 2004/137040 A1 | 6 |
| X | JP 2002-532425 A  (Astra Zeneca AB.),<br>02 October, 2002 (02.10.02),<br>Full text; examples 7, 8<br>& WO 00/35448 A1        & AU 2000/20186 A1<br>& EP 1150677 A1        & KR 2001/93822 A | 1,4,5 |

☒ Further documents are listed in the continuation of Box C.　　　☐ See patent family annex.

| | |
| --- | --- |
| *　　Special categories of cited documents:<br>"A"　document defining the general state of the art which is not considered to be of particular relevance<br>"E"　earlier application or patent but published on or after the international filing date<br>"L"　document which may throw doubts on priority claim(s) or which is cited to establish the publication date of another citation or other special reason (as specified)<br>"O"　document referring to an oral disclosure, use, exhibition or other means<br>"P"　document published prior to the international filing date but later than the priority date claimed | "T"　later document published after the international filing date or priority date and not in conflict with the application but cited to understand the principle or theory underlying the invention<br>"X"　document of particular relevance; the claimed invention cannot be considered novel or cannot be considered to involve an inventive step when the document is taken alone<br>"Y"　document of particular relevance; the claimed invention cannot be considered to involve an inventive step when the document is combined with one or more other such documents, such combination being obvious to a person skilled in the art<br>"&"　document member of the same patent family |

| Date of the actual completion of the international search<br>28 June, 2005 (28.06.05) | Date of mailing of the international search report<br>12 July, 2005 (12.07.05) |
| --- | --- |
| Name and mailing address of the ISA/<br>Japanese Patent Office | Authorized officer |
| Facsimile No. | Telephone No. |

Form PCT/ISA/210 (second sheet) (January 2004)

**INTERNATIONAL SEARCH REPORT**

International application No.

PCT/JP2005/004568

C (Continuation).   DOCUMENTS CONSIDERED TO BE RELEVANT

| Category* | Citation of document, with indication, where appropriate, of the relevant passages | Relevant to claim No. |
|---|---|---|
| X | JP 9-511767 A  (Astra AB.),<br>25 November, 1997 (25.11.97),<br>Full text; examples 1 to 9<br>& WO 96/24375 A1          & AU 9646822 B<br>& EP 754061 A1            & US 6136344 A | 1,4,5 |
| X | JP 11-509829 A  (Merck & Co., Inc.),<br>31 August, 1999 (31.08.99),<br>Full text; example 2<br>& WO 96/19201 A1          & AU 96644726 B<br>& US 558238 A             & EP 801560 A1 | 1,4,5 |

Form PCT/ISA/210 (continuation of second sheet) (January 2004)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 02087622 A